# EUROPÄISCHE PATENTANMELDUNG

(11) **EP 2 679 265 A1**
(43) Veröffentlichungstag der Anmeldung: **01.01.2014**
(21) Anmeldenummer: 12174137.5
(22) Anmeldetag: 28.06.2012
(51) Int. Cl.: A61M 15/00, B65D 47/08

(54) **Kunststoffscharnier**

(71) Anmelder: Pari GmbH, 82319 Starnberg (DE)
(72) Erfinder: Kreutzmann, Vera, 82064 Straßlach Dingharting (DE); Rosenbeiger, Sven, 82319 Starnberg (DE)
(74) Vertreter: Rentsch Partner AG

(57) **Zusammenfassung**

Die Erfindung betrifft ein Kunststoffscharnier (2) zum Wirkverbinden eines Unterteils (3) mit einem Oberteil (4). Das Kunststoffscharnier (2) weist eine Hauptscharnierverbindung (5) auf, welche zwischen dem Unterteil (3) und dem Oberteil (4) angeordnet ist und bezüglich welcher das Oberteil (4) relativ zum Unterteil (3) um eine Hauptachse (16) drehbar ist. Zwei seitlich neben der Hauptscharnierverbindung (15) angeordnete Spannelemente (17) sind so ausgestaltet, dass sie in Offenstellung des Scharniers (2) im aussen liegenden Scharnierbereich in Richtung einer ersten Entformungsrichtung (21) entformbar sind, die in etwa parallel zur Hauptachse (16) angeordnet ist.

## Beschreibung

Die vorliegende Erfindung liegt auf dem Gebiet der Kunststoffscharniere gemäss dem Oberbegriff des unabhängigen Patentanspruchs.

Aus dem Stand der Technik sind Scharniere aus Kunststoff bekannt, die einen Schnappeffekt aufweisen, der durch ihre Geometrie bestimmt wird. Es sind unterschiedliche Funktionsprinzipien für Schnappscharniere bekannt. Praktisch alle basieren auf einer vom Öffnungswinkel abhängigen Deformation bestimmter Teile des Schnappscharniers. Die Schnappscharniere weisen zwei stabile Positionen auf, welche einer Schliessstellung und einer Offenstellung entsprechen. Befinden sich die Scharnierteile zwischen diesen beiden Positionen, kehren sie in aller Regel von selber in die nächste benachbarte Position zurück.

Kostengünstig herzustellende Schnappscharniere aus Kunststoff werden durch Spritzgiessen von Kunststoff einteilig hergestellt. Die Scharniere werden durch Dünnstellen (sog. Filmscharniere) gebildet. Populäre Scharniere weisen eine Hauptscharnierverbindung auf, welche ein erstes und ein zweites Scharnierteil miteinander verbindet. Infolge der Hauptscharnierverbindung drehen die beiden Scharnierteile relativ zueinander auf einer Kreisbahn. Benachbart zu dieser Hauptscharnierverbindung sind eines oder mehrere Federelemente angebracht. Diese sind so angeordnet, dass sie während dem Öffnen, respektive Schliessen des Scharniers gespannt werden und so einen Schnappeffekt bewirken. Die Federelemente werden in der Regel an zwei Seiten durch V-förmig angeordnete, gerade oder gekrümmte Filmscharniere berandet, welche die Anbindung an die Scharnierteile bilden.

Spritzgegossene Schnappscharniere, werden üblicherweise in geöffneter Position hergestellt, da die Scharnierteile ansonsten nicht entformbare Hohlräume bilden. Die Scharnierteile werden dabei in "Achsrichtung", d.h. senkrecht zum geöffneten Scharnier, entformt. In der Regel stellt die Entformung der Scharnierteile keine grösseren Probleme dar. Kleinere Hinterschnitte können meist zwangsentformt werden.

Es gibt Anwendungsbereiche, in denen die aus dem Stand der Technik bekannten Schnappscharniere nicht einfach entformt werden können, da z.B. unterhalb oder oberhalb des Scharniers andere Bestandteile angeordnet sind, die der Entformung im Wege stehen. Ein Anwendungsbereich bilden medizinische Inhalatoren mit einem Inhalatoroberteil aus Kunststoff. Bei diesen Produkten kann es vorkommen, dass eine Entformung aufgrund der Anordnung funktionsrelevanter Öffnungen und Anschlüsse nicht oder nur mit sehr grossem formenbautechnischem Aufwand möglich ist, da komplizierte Anordnungen von Schiebern benötigt werden. Bei Scharnieren ohne Schnappeffekt (sog. tote Scharniere), bei welchen der Scharnierbereich durch ein einfaches Band gebildet wird, stellt sich dieses Problem normalerweise nicht, da die Entformung grundsätzlich wesentlich einfacher ist.

Nachfolgend werden einige Publikationen aus dem Stand der Technik kurz erläutert.

DE3150493 des Erfinders Wilhelm Wiesinger wurde 1982 veröffentlicht und beschreibt ein einteiliges Klappscharnier aus Kunststoff. Bei diesem sind zwei um eine geometrische Hauptachse klappbare Scharnierteile durch Filmscharniere und mindestens ein zwischen diesen angeordnetes Zwischenelement miteinander gelenkig verbunden. Ein Zwischenelement ist durch Filmscharniere eingegrenzt, die gerade oder gekrümmt auseinander verlaufen und schräg zur Hauptachse angeordnet sind. In einer Ausführungsform weisen die Zwischenelemente einen U-förmigen Querschnitt auf. Aus dem Dokument sind keine Hinweise über die Entformbarkeit des Scharniers zu entnehmen.

USD638117**,** respektive USD652908**,** beide derselben Anmelderin, wurden am 17.05.2011, respektive 24.01.2012 publiziert und zeigen Designs von Inhalatoren mit einem in etwa zylindrischen Unterteil und einem als Deckel dienenden Oberteil. Ein Scharnier, welches das Unterteil mit dem Oberteil verbindet und aus mehreren Materialkomponenten besteht, ist oberhalb eines seitlich vorstehenden Flansches angeordnet. In Realität weist das Produkt zwei Filmscharniere aus einer harten Materialkomponente auf. Dazwischen ist eine weichere Materialkomponente aus einem zweiten Material angeordnet, welche eine Art Spannband bildet.

WO2005/032630 derselben Anmelderin wurde am 14.04.2005 veröffentlicht und beschreibt eine Inhaltionstherapievorrichtung mit einer Verneblerkammer, in der ein Aerosolerzeuger angeordnet ist, der ein Aerosol in die Verneblerkammer abgibt. An einer Öffnung der Verneblerkammer ist ein Ventil angeordnet, das einen Ventilsitz, ein elastisches Ventilelement und eine Ventilelementpositionierungseinrichtung umfasst. Die Inhalationstherapievorrichtung umfasst einen Deckel, der über ein sog. Filmscharnier verbunden ist.

Die Ventilelementpositionierungseinrichtung kann von einer ersten in eine zweite Position gebracht werden, so dass sie das Ventilelement an dem Ventilsitz derart positioniert, dass in der ersten Position das Ventilelement in einem strömungsfreien Zustand mit einer Vorspannung auf dem Ventilsitz positioniert wird und in der zweiten Position von dem Ventilsitz beabstandet ist.

EP1963195 der Seaquist Closures Inc. wurde 2005 erstmals publiziert und beschreibt einen Verschluss für ein Behältnis. Der Verschluss weist ein integral mit den Verschlussteilen ausgebildetes Scharnier auf, welches in den Aussenbereichen Dünnstellen aufweist, die zur Reduktion der Spannungen im Scharnier dienen.

EP0933304 der Terxo AG wurde 1999 erstmals publiziert und betrifft ein Kunststoffschnappscharnierverschluss. Dieser weist ein Unterteil und eine Kappe auf, die über ein Schnappscharnier miteinander verbunden sind. Um ein Reissen an den seitlichen Rändern zu verhindern, wird eine sich über das Scharnier erstreckende, in Höhe oder Wandstärke variierende Auswölbung vorgesehen, die mittig druckelastisch und seitlich zugelastisch ausgestaltet ist. Allerdings erweist sich dieses Scharnier als nicht unproblematisch bei der Entformung.

Eine Aufgabe der Erfindung besteht darin ein Kunststoffscharnier zu zeigen, welches einfach entformbar ist.

Diese Aufgabe wird durch das in den unabhängigen Patentansprüchen definierte Scharnier gelöst.

In einer Ausführungsform weist das erfindungsgemässe Scharnier eine Hauptscharnierverbindung auf, welche ein Unterteil mit einem Oberteil wirkverbindet. Auf jeder Seite dieser Hauptscharnierverbindung ist jeweils ein Spannelement angeordnet, das beim Öffnen, respektive Schliessen, des Oberteils gegenüber dem Unterteil kontrolliert deformiert wird. Diese Spannelemente sind mit Vorteil so ausgestaltet, dass sie zu einem Schnappeffekt beitragen. Der Schnappeffekt bewirkt, dass das Oberteil relativ zum Unterteil in einer definierten Position verbleibt. Die Spannelemente sind so ausgestaltet, dass sie in Querrichtung, d.h. parallel zur Achse der Hauptscharnierverbindung, zumindest im aussen liegenden Scharnierbereich keinen Hinterschnitt bilden, der nicht entformbar ist. Im innen liegenden Scharnierbereicht kann die Entformung bei geöffneten Deckelteilen senkrecht zur Scharnierebene vorgenommen werden.

In einer Ausführungsform betrifft die Erfindung ein Kunststoffscharnier zum Wirkverbinden eines Unterteils mit einem Oberteil. Das Kunststoffscharnier weist eine Hauptscharnierverbindung auf, welche zwischen dem Unterteil und dem Oberteil angeordnet ist und bezüglich welcher das Oberteil relativ zum Unterteil um eine Hauptachse rotierbar angeordnet ist. Zwei seitlich neben der Hauptscharnierverbindung und zwischen dem Oberteil und dem Unterteil angeordnete Spannelemente sind so ausgestaltet, dass sie in Offenstellung des Scharniers im aussen liegenden Scharnierbereich in Richtung einer ersten Entformungsrichtung entformbar sind. Die erste Entformungsrichtung ist mit Vorteil in etwa parallel zur Hauptachse angeordnet. Die Spannelemente können auf der Scharnierinnenseite so ausgestaltet sind, dass sie in einer zweiten Entformungsrichtung entformbar sind. Die zweite Entformungsrichtung ist mit Vorteil im Wesentlichen senkrecht zur ersten Entformungsrichtung angeordnet. Die Spannelemente können über mindestens je ein Filmscharnier mit dem Unterteil und dem Oberteil wirkverbunden sein. Die Filmscharniere sind in der Regel V-förmig zueinander angeordnet. Die Filmscharniere können entlang des Unterteils, respektive des Oberteils angeordnet sein. Die Spannelemente weisen mit Vorteil ein in Richtung der ersten Entformungsrichtung einen U-förmigen Querschnitt auf, der sich zur Hauptscharnierverbindung hin verjüngt. Die Spannelemente können eine nach aussen hin zunehmende Erhöhung aufweisen, welche z.B. entgegen dem Verlauf der Filmscharniere ausgestaltet ist. Die Erhöhung kann eine grössere Wandstärke aufweisen, als die Wandstärke der Filmscharniere.

Anhand der in den nachfolgenden Figuren gezeigten Ausführungsbeispiele und der dazugehörigen Beschreibung werden Aspekte der Erfindung näher erläutert. Es zeigen:
- Fig. 1: eine erste Ausführungsform eines Inhalators mit einem Scharnier gemäss der Erfindung;
- Fig. 2: den Inhalator mit dem Scharnier gemäss Figur 1 in einer Draufsicht;
- Fig. 3: Detail D gemäss Figur 1;
- Fig. 4: Detail E gemäss Figur 2;
- Fig. 5: den Inhalator mit dem Scharnier in einer perspektivischen Darstellung von schräg vorne und oben;
- Fig. 6: den Inhalator mit dem Scharnier in einer perspektivischen Darstellung von schräg hinten und oben.

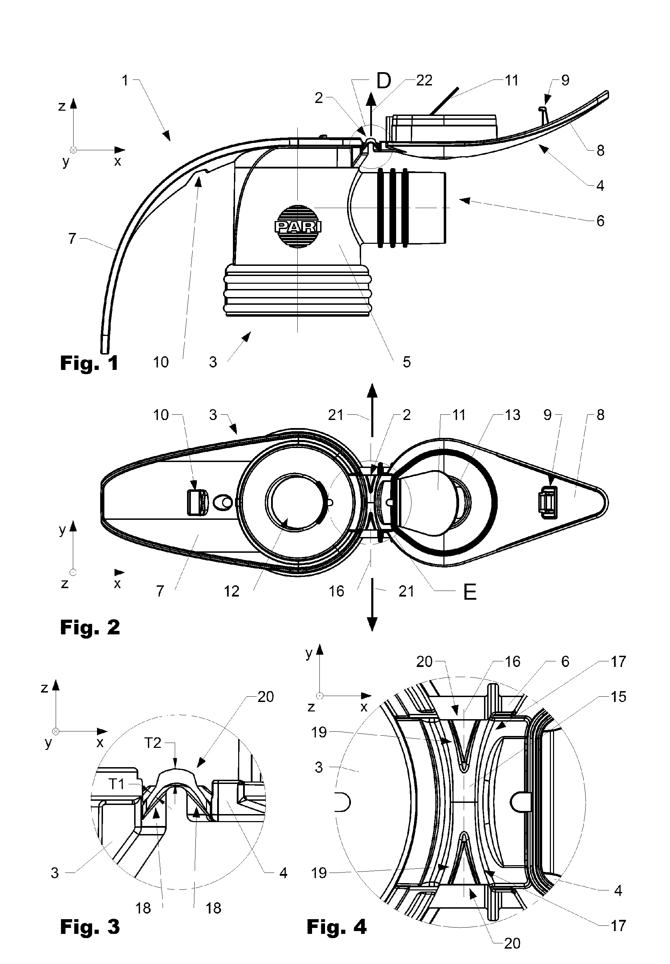

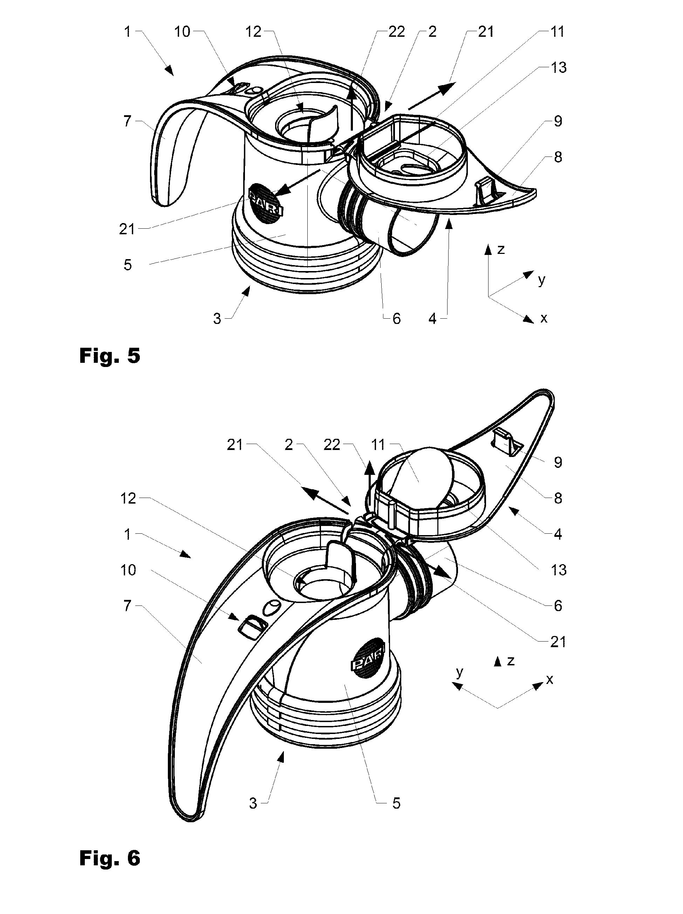

**Figur 1** zeigt einen Inhalatorkopf (fortan Inhalator) 1 mit einem erfindungsgemässen Scharnier 2 in einer Seitenansicht. **Figur 2** zeigt den Inhalator 1 mit dem Scharnier 2 in einer Draufsicht. **Figur 3** zeigt Detail D gemäss Figur 1 in einer vergrösserten Darstellung. **Figur 4** zeigt Detail E in einer gemäss Figur 2 in einer vergrösserten Darstellung. **Figur 5** zeigt den Inhalatorkopf 1 mit dem Scharnier 2 in einer perspektivischen Darstellung von schräg vorne und oben und **Figur 6** zeigt den Inhalatorkopf 1 mit dem Scharnier 2 in einer perspektivischen Darstellung von schräg hinten und oben.

Der Inhalator 1 weist ein Unterteil 3 und ein Oberteil (Deckel) 4 auf, welche über das Scharnier 2 miteinander wirkverbunden sind. In den Figuren ist der Deckel 4 und das Scharnier 2 in geöffneter Position gezeigt. In dieser Position werden sie durch Spritzgiessen mindestens einer ersten Materialkomponente hergestellt.

Der Unterteil 3 weist einen in etwa zylindrischen Grundkörper 5 auf an welchem ein seitlich vorstehender Anschlussstutzen 6 für eine Inhalationsmaske (nicht näher dargestellt) ausgebildet ist. Der Anschlussstutzen 6 ist unterhalb des Scharniers 2 angeordnet. Aus diesem Grund ist eine Entformung des Scharniers in dieser Richtung nicht möglich. Der Grundkörper 5 weist einen nach unten geschwungenen Griff 7 auf, der in Schliessstellung des Deckels 4 mit einem an diesem ausgebildeten Vorsprung 8 korrespondiert. Der Deckel 4 weist zudem einen Schnapphacken 9 auf, der in Schliessstellung in einer Einrastöffnung 10 einrastet und den Deckel so in Schliessstellung (nicht näher dargestellt) fixiert.

Am Deckel 4 ist eine Ventillasche 11 aus einem flexiblen Material hergestellt. In der gezeigten Ausführungsform ist die Ventillasche 11 durch Spritzgiessen aus einer zweiten Materialkomponente hergestellt und dabei vorzugsweise integral mit der ersten Materialkomponente des Grundkörpers wirkverbunden werden. Die Ventillasche 11 muss in Schliessstellung des Deckels 4 gegenüber einer Durchgangsöffnung 12 des Unterteils 3, respektive einem umlaufenden Dichtrahmen 13 des Oberteils 4 sehr genau positioniert werden, was durch das erfindungsgemässe Scharnier 2 in Kombination mit dem Schnapphacken 9 und der Einrastöffnung 10 unterstützend gewährleistet wird.

Wie u. a. in den vergrösserten Detailansichten gemäss den **Figuren 3** und **4** hervorgeht, weist das Scharnier 2 eine zentrale Hauptscharnierverbindung 15 auf, welche den Unterteil 3 mit dem Oberteil 4 um eine Hauptachse 16 - schematisch als gestrichelte Linie dargestellt - gelenkig drehbar verbindet. Auf beiden Seiten der Hauptscharnierverbindung 15 sind je ein Spannelement 17 angeordnet, welche ebenfalls den Unterteil 3 mit dem Oberteil 4 wirkverbinden. Die Spannelemente 17 sind auf einer anderen Kreisbahn angeordnet und werden dadurch beim Öffnen, respektive Schliessen des Deckels 4 gegenüber dem Unterteil 3 über einen Totpunkt hinweg gespannt. Die Spannelemente 17 weisen in der Draufsicht (vgl. **Figur 2****,** resp. **Figur 4**) gesehen eine in etwa V-förmige Form auf, welche entlang dem Unterteil 3 und dem Oberteil 4 (Randzonen 18) von der Hauptscharnierverbindung 15 her nach aussen (y-Richtung) und unten (z-Richtung) auseinanderstreben. Die Randzonen können als Filmscharniere 18 ausgestaltet sein.

Wie in der vergrösserten Detailansichten gemäss den **Figuren 3** und **4** zu erkennen ist, weisen die Spannelemente 17 des erfindungsgemässen Scharniers 2 im Unterschied zu herkömmlichen Scharnieren, in Aussenzonen 19 Erhöhung 20 auf, welche in Offenstellung entgegen dem nach unten verlaufenden Trend der Randzonen 18, in der Darstellung nach oben ausgebildet sind. Die Spannelemente 17 der gezeigten Ausführungsform sind in Offenstellung so ausgestaltet, dass sie auf der Aussenseite in einer ersten Entformungsrichtung 21 (hier y-Richtung) keinen nicht entformbaren Hinterschnitt bilden. Auf der Scharnierinnenseite wird das Scharnier 2 vorzugsweise in Richtung einer zweiten Entformungsrichtung 22 (hier z-Richtung) entformt. Die Erhöhungen 20 können eine, im Vergleich zur Randzonen 18, welche eine erste Wandstärke T1 aufweisen, eine zweite, grössere Wandstärke T2 aufweisen. Diese kann zum Einstellen eines Schnappeffektes des Scharniers 2 dienen. Wie in der vergrösserten Draufsicht gemäss **Figur 4** zu erkennen ist, enden die Erhöhungen 20 auf beiden Seiten der Hauptscharnierverbindung 15.

### LISTE DER BEZUGSZEICHEN

- 1: Inhalatorkopf
- 2: Scharnier
- 3: Unterteil
- 4: Oberteil (Deckel)
- 5: Grundkörper
- 6: Anschlussstutzen
- 7: Griff
- 8: Vorsprung
- 9: Schnapphacken
- 10: Einrastöffnung
- 11: Ventillasche
- 12: Durchgangsöffnung
- 13: Dichtrahmen
- 15: Hauptscharnierverbindung
- 16: Hauptachse
- 17: Spannelement
- 18: Randzone / Filmscharnier
- 19: Aussenzone
- 20: Erhöhung
- 21: erste Entformungsrichtung
- 22: zweite Entformungsrichtung

## Patentansprüche

1. Kunststoffscharnier (2) zum Wirkverbinden eines Unterteils (3) mit einem Oberteil (4), wobei
a. das Kunststoffscharnier (2) eine Hauptscharnierverbindung (15) aufweist, welche zwischen dem Unterteil (3) und dem Oberteil (4) angeordnet ist und bezüglich welcher das Oberteil (4) relativ zum Unterteil (3) um eine Hauptachse (16) rotierbar angeordnet ist und
b. zwei seitlich neben der Hauptscharnierverbindung (15) angeordnete Spannelemente (17), welche zwischen dem Oberteil (4) und dem Unterteil (3) anordnet sind und so ausgestaltet sind, dass sie in Offenstellung des Scharniers (2) im aussen liegenden Scharnierbereich in Richtung einer ersten Entformungsrichtung (21) entformbar sind, die in etwa parallel zur Hauptachse (16) angeordnet ist.

2. Kunststoffscharnier (2) gemäss Patentanspruch 1, **dadurch gekennzeichnet, dass** die Spannelemente (17) auf der Scharnierinnenseite so ausgestaltet sind, dass sie in einer zweiten Entformungsrichtung (22) entformbar sind.

3. Kunststoffscharnier (2) gemäss Patentanspruch 2, **dadurch gekennzeichnet, dass** die zweite Entformungsrichtung (22) im Wesentlichen senkrecht zur ersten Entformungsrichtung (21) steht.

4. Kunststoffscharnier (2) gemäss einem der vorangehenden Patentansprüche, **dadurch gekennzeichnet, dass** die Spannelemente (17) über mindestens je ein Filmscharnier mit dem Unterteil (3) und dem Oberteil (4) wirkverbunden sind.

5. Kunststoffscharnier (2) gemäss Patentanspruch 2, **dadurch gekennzeichnet, dass** die Filmscharniere V-förmig zueinander angeordnet sind.

6. Kunststoffscharnier (2) gemäss Patentanspruch 2 oder 5, **dadurch gekennzeichnet, dass** die Filmscharniere entlang des Unterteils (3), respektive des Oberteils (4) angeordnet sind.

7. Kunststoffscharnier (2) gemäss einem der vorangehenden Patentansprüche, **dadurch gekennzeichnet, dass** die Spannelemente (17) in Richtung der ersten Entformungsrichtung (21) einen U-förmigen Querschnitt aufweisen, der sich zur Hauptscharnierverbindung (15) hin verjüngt.

8. Kunststoffscharnier (2) gemäss einem der vorangehenden Patentansprüche, **dadurch gekennzeichnet, dass** die Spannelemente (17) eine nach aussen hin zunehmende Erhöhung (20) aufweist, welche entgegen dem Verlauf der Filmscharniere (18) ausgestaltet ist.

9. Kunststoffscharnier (2) gemäss Patentanspruch 8, **dadurch gekennzeichnet, dass** die Erhöhung (20) eine grössere Wandstärke (T2) aufweist, als die Wandstärke (T1) der Filmscharniere (18).

10. Inhalator mit einem Kunststoffscharnier gemäss einem der vorangehenden Patentansprüche.

11. Inhalator gemäss Patentanspruch 10, **dadurch gekennzeichnet, dass** unterhalb des Scharniers (2) ein Anschlussstutzen (6) angeordnet ist.
